# FASCICULE DE BREVET EUROPEEN

(11) **EP 2 157 938 B1**
(45) Date de publication et mention de la délivrance du brevet: **01.02.2012**
(21) Numéro de dépôt: 07788991.3
(22) Date de dépôt: 12.06.2007
(51) Int. Cl.: A61F 2/44

(54) **CAGE EXPANSIVE POUR CHIRURGIE VERTÉBRALE DU TYPE FUSION INTERSOMATIQUE LOMBAIRE PAR VOIE POSTÉRIEURE TRANSFORAMINALE**
EXPANDIERBARER KÄFIG FÜR WIRBELOPERATIONEN MIT LUMBALER INTERSOMATISCHER FUSION AUF TRANSFORAMINALEM POSTERIOREM WEG
EXPANDABLE CAGE FOR VERTEBRAL SURGERY INVOLVING LUMBAR INTERSOMATIC FUSION BY A TRANSFORAMINAL POSTERIOR APPROACH

(43) Date de publication de la demande: 03.03.2010
(73) Titulaire: Ros Guillen, Francisco, 13700 Marignane (FR); Schaumburg, Gérald, 13110 Port de Bouc (FR); Attia, David, 26200 Montélimar (FR)
(72) Inventeur: ATTIA, David, F-26200 Montelimar (FR)
(74) Mandataire: Novagraaf Technologies
(86) Numéro de dépôt international: PCT/FR2007/051425
(87) Numéro de publication internationale: WO 2008/155472

(56) Documents cités:
- EP-A- 1 415 622
- US-A1- 2006 129 244
- US-A1- 2006 287 725

## Description

La présente invention concerne un implant appelé cage utilisé en chirurgie vertébrale pour une opération de fusion entre deux corps vertébraux d'une colonne vertébrale, introduit entre les corps vertébraux par une voie dite postérieure transforaminale. Une telle opération est connue sous le nom de TLIF (transforaminal lumbar interbody fusion).

Un implant de ce type est décrit, par exemple, dans la demande de brevet FR2861582. Il présente un profil externe courbe en forme de haricot adapté à l'insertion par voie postérieure transforaminale entre deux vertèbres. Autrement dit, l'axe de l'implant, suivant lequel il est introduit dans l'espace discal, est courbe. Cet implant est pourvu d'une cavité intérieure de réception de greffe osseuse, naturelle ou synthétique, ce qui explique le nom de cage qui est également donné à cet implant. Cette cage est ouverte à chacune de ses extrémités inférieure et supérieure pour permettre la mise en contact de la greffe avec les corps vertébraux respectifs, de manière à réaliser une fusion définitive entre ces corps vertébraux.

L'arthrodèse intersomatique est réalisée par un abord postéro-latéral après résection de tout ou partie d'un massif articulaire postérieur, à travers un interstice entre le fourreau dural et la racine sus jacente. Après introduction, l'implant progresse vers la partie antérieure de l'espace discal en tournant de 90° entre les corps vertébraux pour se trouver dans sa position finale, s'étendant transversalement dans l'espace discal.

L'implant est pré-rempli de greffons osseux avant insertion. En effet, une fois installé dans sa position finale, l'implant n'est plus accessible.

L'insertion de tels implants dans des disques souvent très pincés nécessite la mise en place préalable de vis pédiculaires permettant d'ouvrir l'espace discal. De plus, une difficulté supplémentaire est due la difficulté de faire tourner de 90° l'implant dans un tel espace serré tout en lui imprégnant un mouvement de rotation, eu égard au fait que les vis n'ouvrent que la partie postérieure de l'espace discal.

Il a été proposé des implants expansifs permettant l'ouverture de l'espace discal de manière plus efficace qu'avec une vis pédiculaire. Ainsi, la demande de brevet US 2006/0129244 décrit un dispositif écarteur adapté à l'insertion entre deux corps vertébraux comprenant deux plaques et un membre écarteur coulissant entre ces deux plaques suivant l'axe de l'implant de manière à écarter ces deux plaques entre une position non écartée et une position écartée.

Un tel dispositif permet en effet une meilleure ouverture de l'espace discal. Toutefois, il reste très difficile voire impossible d'introduire des greffons osseux en utilisant un tel dispositif, en particulier au niveau de la partie antérieure de l'implant.

Un des objets de la présente invention est de proposer un implant pouvant être inséré par voie antérieure transforaminale entre deux vertèbres adjacentes et permettant une introduction aisée de greffons osseux.

À cet effet, l'invention concerne un implant chirurgical de type cage intersomatique pour la fusion de corps vertébraux par abord transforaminal comprenant un corps à profil courbe destiné à être implanté entre les plateaux vertébraux de deux vertèbres adjacentes et au moins un logement ouvert en haut et en bas pour recevoir un élément de greffe osseuse, caractérisé en ce que ledit corps à profil courbe comprend une partie postérieure de manipulation et une partie antérieure d'attaque, ladite partie antérieure d'attaque étant constituée d'une languette inférieure et d'une languette supérieure adaptées à recevoir entre elles un élément écarteur pouvant coulisser entre une position d'introduction dans laquelle il se trouve proche de ladite partie postérieure de manipulation et une position d'écartement dans laquelle il se trouve proche de l'extrémité de ladite partie antérieure d'attaque dont il augmente l'épaisseur par écartement des languettes, ledit élément écarteur comprenant un logement ouvert en haut et en bas adapté à recevoir ledit élément de greffe osseuse dans ladite position d'introduction et à l'amener au niveau d'une partie antérieure desdits plateaux vertébraux dans ladite position d'écartement.

Par extrémité d'attaque, on entend l'extrémité qui est destinée à être insérée entre les corps vertébraux, tandis que par extrémité de manipulation, on entend l'extrémité opposée, sur laquelle sont exercés les efforts destinés à faire pénétrer le dispositif entre lesdits plateaux vertébraux.

Ledit élément écarteur en coulissant permet en même temps l'expansion de la cage et l'amenée de l'élément de greffe osseuse vers les zones antérieures difficilement accessibles de l'espace intervertébral.

Avantageusement, ledit élément écarteur comprend deux cloisons transversales délimitant ledit logement adapté à recevoir ledit élément de greffe osseuse, lesdites cloisons ayant une hauteur correspondant à la hauteur dudit implant en position d'écartement et une largeur correspondant à la largeur d'une ouverture centrale ménagée le long de chacune des languettes.

De la sorte, l'élément écarteur forme un tiroir coulissant entre les languettes délimité par des cloisons ayant toujours la même hauteur et qui peut donc transporter le greffon vers la partie antérieure de l'implant.

Avantageusement, au moins une desdites languettes est pourvue de moyens de verrouillage par enclipsage adaptés à verrouiller ledit élément écarteur dans ladite position d'écartement.

Avantageusement, ledit corps à profil courbe est en forme de haricot.

Avantageusement, lesdites languettes sont pourvues sur leurs faces extérieures de nervures adaptées à venir en prise avec les plateaux des corps vertébraux.

Avantageusement, ladite extrémité de manipulation est pourvue d'un orifice permettant la manipulation dudit élément écarteur et l'introduction d'un second élément de greffe osseuse lorsque ledit élément écarteur se trouve dans ladite position d'écartement.

La présente invention sera mieux comprise à la lecture de la description d'un exemple non limitatif de réalisation qui suit, en référence aux dessins annexés où :
la figure 1 est une vue schématique en perspective d'un implant selon la présente invention en position d'insertion,
la figure 2 est une vue similaire à celle de la figure 1 en position d'écartement,
la figure 3 est une vue en plan de dessous d'un corps principal du dispositif des figures 1 et 2,
la figure 4 est une vue en plan de dessous d'un tiroir écarteur du dispositif des figures 1 et 2, et
la figure 5 est une vue en plan de dessous du dispositif de la figure 2.

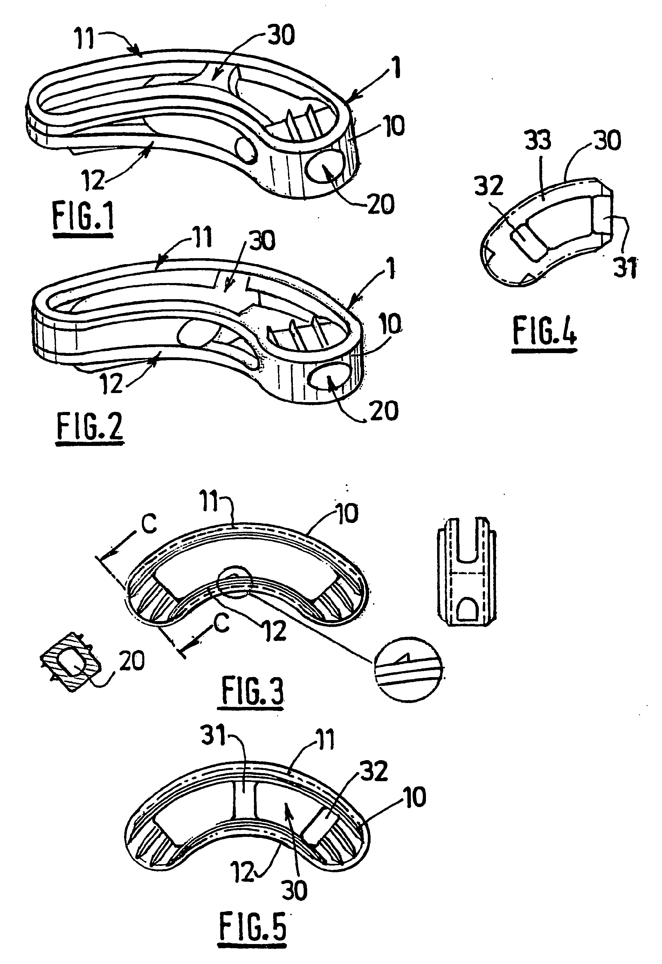

En référence aux figures 1 à 5, un implant chirurgical 1 est de type cage intersomatique pour la fusion de corps vertébraux par abord transforaminal.

L'implant 1 est en matériau élastique biocompatible. Avantageusement, l'implant est en PEEK. Alternativement, il peut être en métal, tel que du titane, ou en matériau bio-résorbable tel que du PLLA.

Globalement, cet implant 1 comprend un corps 10 en forme de haricot destiné à être implanté entre les plateaux de deux vertèbres adjacentes, un élément écarteur 30 formant tiroir adapté à être rempli de greffon osseux coulissant à l'intérieur de ce corps, suivant l'axe courbe de ce dernier entre une position d'introduction de l'implant représentée sur la figure 1 -dans laquelle l'élément écarteur se trouve au niveau d'une zone postérieure du corps 10- et une position d'écartement représentée sur la figure 2 -dans laquelle l'élément écarteur se trouve au niveau d'une extrémité antérieure du corps 10.

Le corps 10, formant un seul bloc venant de moulage ou d'usinage, comprend une partie postérieure de manipulation et une partie antérieure d'attaque.

La partie postérieure de manipulation est en forme, globalement, demi-anneau fermé en haut et en bas par des cloisons supérieure et inférieure pourvues sur leurs faces extérieures de nervures s'étendant selon l'axe de l'implant. Ces nervures sont adaptées à venir en prise avec les plateaux des vertèbres. Par ailleurs, cette partie annulaire est pourvue au niveau de son extrémité postérieure d'un orifice 20 permettant la manipulation du tiroir écarteur 30 et le remplissage de la partie postérieure de la cage après expansion de cette dernière.

La partie antérieure d'attaque, qui forme en réalité la majeure partie de l'implant, est constituée d'une languette supérieure 11 et d'une languette inférieure 12 s'étendant respectivement des extrémités supérieure et inférieure de la partie postérieure de manipulation. Ces languettes 11 et 12, de formes identiques, sont creuses en leurs centres, de sorte qu'elles sont en fait constituées de bords latéraux s'étendant suivant l'axe de l'implant et d'une flasque d'extrémité de forme identique à la paroi ménagée au niveau de l'extrémité opposée, au niveau de la partie postérieure de manipulation. Comme pour cette paroi, des nervures sont ménagées sur la face extérieure de cette flasque. Les nervures de la flasque et de la paroi se trouvant en regard des bords latéraux sont prolongées sur ces derniers de manière à former deux nervures latérales s'étendant suivant l'axe courbe de l'implant et adaptées à venir en prise avec le plateau du corps vertébral correspondant.

Des éléments de verrouillage par enclipsage sont ménagés sous les bords latéraux. Ces éléments de verrouillage, dont l'un est représenté sur la figure 3, présentent, de manière classique, un bord en pente douce que l'élément écarteur vient écraser lorsqu'il coulisse de la position d'introduction vers la position d'écartement et un bord vertical qui se redresse élastiquement derrière l'élément écarteur lorsque ce dernier le libère. L'élément de verrouillage est positionné de manière à être libéré lorsque l'extrémité de l'élément écarteur se trouve au niveau de l'extrémité des languettes, ce qui permet ainsi le verrouillage de l'implant dans la position d'écartement.

Des éléments de guidage sont ménagés sur les bords latéraux pour coopérer avec des éléments de guidage correspondants ménagés sur l'élément écarteur de manière à ce que ce dernier coulisse à l'intérieur du corps 10 suivant l'axe de ce dernier.

L'élément écarteur 30 comprend deux bords latéraux 33 ayant la même courbure et le même écartement que les bords latéraux correspondants du corps principal 10. Ces deux bords se rejoignent au niveau d'une extrémité antérieure par une extrémité courbe de forme adaptée à prolonger les flasques d'extrémités des languettes du corps 10 en position d'écartement, de manière à reproduire une forme similaire à celle de la partie postérieure de manipulation de ce corps. Par ailleurs, la hauteur dudit élément écarteur ajoutée à la hauteur des languettes correspond précisément à la hauteur de la partie postérieure de manipulation. De la sorte, en position d'écartement, l'implant 1 a une hauteur identique sur toute sa longueur et a un profil en haricot globalement symétrique correspondant à celui des implants selon l'art antérieur.

L'élément écarteur 30 comprend un logement ouvert en haut et en bas adapté à recevoir un greffon osseux. Deux cloisons transversales 31 et 32 sont ménagées l'une au niveau de l'extrémité postérieure de l'élément écarteur 30 et l'autre à proximité de son extrémité antérieure, légèrement en retrait de la partie antérieure courbe. Ces cloisons ont une hauteur identique à la hauteur de la partie postérieure de manipulation, c'est-à-dire à la hauteur dudit implant en position d'écartement. Par ailleurs, elles ont une largeur correspondant à l'écartement entre les bords latéraux des languettes, c'est-à-dire à la largeur de l'ouverture centrale ménagée le long de chacune des languettes.

L'implant, dans la position d'introduction représentée sur la figure 1 dans laquelle l'élément écarteur 30 se trouve en butée contre la partie postérieure de manipulation du corps 10, est pré-rempli de greffons osseux introduit dans l'espace délimité par les parois 31 et 32 et, latéralement, par les parois constituées par l'empilement des bords latéraux des languettes et de l'élément écarteur. Après insertion de l'implant entre les corps vertébraux l'élément écarteur est ensuite poussé de manière à coulisser entre les languettes. Ceci a pour effet d'écarter les languettes l'une de l'autre. La partie avant de l'élément écarteur règle l'écartement des languettes à une hauteur égale à celle des parois 31 et 32 au fur et à mesure de sa progression, de sorte que le greffon se trouve toujours dans un compartiment délimité par des parois de hauteur identique et ne risque pas de s'échapper. Autrement dit, l'élément écarteur forme un tiroir qui amène le greffon osseux au niveau de l'extrémité antérieure de l'implant. Une fois que l'extrémité antérieure du tiroir arrive au niveau des extrémités antérieures des languettes, les moyens de verrouillage se déclenchent et immobilisent l'implant dans la position d'écartement représentée sur la figure 2. Le logement délimité entre la paroi 31 et la partie postérieure de l'implant pourra être si besoin alors à son tour comblé de greffe.

## Revendications

1. Implant chirurgical (1) de type cage intersomatique pour la fusion de corps vertébraux par abord transforaminal comprenant un corps à profil courbe destiné à être implanté entre les plateaux vertébraux de deux vertèbres adjacentes et au moins un logement ouvert en haut et en bas pour recevoir un élément de greffe osseuse, **caractérisé en ce que** ledit corps à profil courbe comprend une partie postérieure de manipulation et une partie antérieure d'attaque, ladite partie antérieure d'attaque étant constituée d'une languette inférieure (12) et d'une languette supérieure (11) adaptées à recevoir entre elles un élément écarteur (30) pouvant coulisser entre une position d'introduction dans laquelle il se trouve proche de ladite partie postérieure de manipulation et une position d'écartement dans laquelle il se trouve proche de une l'extrémité de ladite partie antérieure d'attaque dont il augmente l'épaisseur par écartement des languettes (11, 12) ledit élément écarteur comprenant un logement ouvert en haut et en bas adapté à recevoir ledit élément de greffe osseuse dans ladite position d'introduction et à l'amener au niveau d'une partie antérieure desdits plateaux vertébraux dans ladite position d'écartement.

2. Implant chirurgical (1) selon la revendication 1, **caractérisé en ce que** ledit élément écarteur (30) comprend deux cloisons transversales (31, 32) délimitant ledit logement adapté à recevoir ledit élément de greffe osseuse, lesdites cloisons (31, 32) ayant une hauteur correspondant à la hauteur dudit implant (1) en position d'écartement et une largeur correspondant à la largeur d'une ouverture centrale ménagée le long de chacune des languettes (11, 12).

3. Implant (1) selon la revendication 1 ou 2, **caractérisé en ce qu'**au moins une desdites languettes (11, 12) est pourvue de moyens de verrouillage par enclipsage adaptés à verrouiller ledit élément écarteur dans ladite position d'écartement.

4. Implant (1) selon l'une quelconque des revendications précédentes, **caractérisé en ce que** ledit corps à profil courbe est en forme de haricot.

5. Implant (1) selon l'une quelconque des revendications précédentes, **caractérisé en ce que** lesdites languettes (11, 12) sont pourvues sur leurs faces extérieures de nervures adaptées à venir en prise avec les plateaux des .

6. Implant (1) selon l'une quelconque des revendications précédentes, **caractérisé en ce que** ladite partie posterieure de manipulation est pourvue d'un orifice (20) permettant la manipulation dudit élément écarteur et l'introduction d'un second élément de greffe osseuse lorsque ledit élément écarteur se trouve dans ladite position d'écartement.

## Claims

1. An intersomatic cage type surgical implant (1) for fusion of vertebral bodies using a transforaminal approach, comprising a body with a curved profile that is designed to be implanted between vertebral plates of two adjacent vertebrae and at least a housing open on top and bottom in order to receive a bone graft member, wherein said curved profile body comprises a posterior manipulation part and an anterior leading part, said anterior leading part being composed of a lower tongue (12) and of an upper tongue (11) that are designed to receive an expanding element (30) therebetween that can slide between an insertion position, in which it is situated near said posterior manipulation part, and an expanding position, in which it is situated near an end of said anterior leading part, thus increasing the thickness of the latter by spacing the tongues (11, 12) apart, said expanding element comprising a housing that is open on top and bottom adapted to receive said bone graft member in said insertion position and to bring it at an anterior part of said vertebral plates in said expanding position.

2. The surgical implant (1) according to claim 1, wherein said expanding element (30) comprises two transversal walls (31, 32) delimiting said housing adapted to receive said bone graft member, said walls (31, 32) having a height corresponding to the height of said implant (1) in the expanding position and a width corresponding to the width of a central opening provided along each of the tongues (11, 12).

3. The implant (1) according to claim 1 or 2, wherein at least one of said tongues (11, 12) is provided with locking means by clipping adapted to lock said expanding element in said expanding position.

4. The implant (1) according to any one of the preceding claims, wherein said curved profile body is bean shaped.

5. The implant (1) according to any one of the preceding claims, wherein said tongues (11, 12) are provided on their external sides with ribs adapted to engage the plates of the vertebrae.

6. The implant (1) according to any one of the preceding claims, wherein said posterior manipulation part is provided with an opening (20) through which said expanding element can be manipulated and a second bone graft member can be inserted when said expanding element is in said expanding position.

## Patentansprüche

1. Chirurgisches Implantat (1) des intersomatischen Cage-Typs zur transforaminalen Fusion von Wirbelkörpern, umfassend einen Körper mit gekrümmtem Profil, der zwischen Wirbelplatten von zwei benachbarten Wirbeln implantiert werden soll, und mindestens einen oben und unten offenen Ausschnitt zum Aufnehmen eines Knochentransplantatelements, **dadurch gekennzeichnet, dass** der besagte Körper mit gekrümmtem Profil einen hinteren Handhabungsteil und einen vorderen Vorstoßteil umfasst, wobei der besagte vordere Vorstoßteil aus einer unteren Zunge (12) und einer oberen Zunge (11) besteht, die zum Aufnehmen eines Spreizerelements (30) zwischen ihnen eingerichtet sind, das zwischen einer Einführungsstellung, in der es sich in der Nähe des besagten hinteren Handhabungsteils befindet, und einer Spreizstellung, in der es sich in der Nähe eines Endes des besagten vorderen Vorstoßteils befindet, gleiten kann, wodurch es die Dicke durch Spreizen der Zungen (11, 12) erhöht, wobei das besagte Spreizerelement einen oben und unten offenen Ausschnitt umfasst, der in der besagten Einführungsstellung zum Aufnehmen des besagten Knochentransplantatelements und in der besagten Spreizstellung zum Bringen dieses auf Höhe eines vorderen Teils der besagten Wirbelplatten eingerichtet ist.

2. Chirurgisches Implantat (1) nach Anspruch 1, **dadurch gekennzeichnet, dass** das besagte Spreizerelement (30) zwei Querwände (31, 32) umfasst, die den besagten Ausschnitt, der zum Aufnehmen des besagten Knochentransplantatelements eingerichtet ist, abgrenzen, wobei die besagten Wände (31, 32) eine Höhe, die der Höhe des besagten Implantats (1) in der Spreizstellung entspricht, und eine Breite aufweisen, die der Breite einer zentralen Öffnung entspricht, die entlang jeder der Zungen (11, 12) vorgesehen ist.

3. Implantat (1) nach Anspruch 1 oder 2, **dadurch gekennzeichnet, dass** mindestens eine der besagten Zungen (11, 12) mit Mitteln zur Arretierung mittels Aufklipsen umfasst, die zum Arretieren des besagten Spreizerelements in der besagten Spreizstellung eingerichtet sind.

4. Implantat (1) nach einem der vorhergehenden Ansprüche, **dadurch gekennzeichnet, dass** der besagte Körper mit gekrümmtem Profil die Form einer Bohne hat.

5. Implantat (1) nach einem der vorhergehenden Ansprüche, **dadurch gekennzeichnet, dass** die besagten Zungen (11, 12) auf ihren Außenflächen mit Rippen versehen sind, die zum Ineingriffkommen mit den Wirbelplatten eingerichtet sind.

6. Implantat (1) nach einem der vorhergehenden Ansprüche, **dadurch gekennzeichnet, dass** der besagte hintere Handhabungsteil mit einer Öffnung (20) versehen ist, die die Handhabung des besagten Spreizerelements und die Einführung eines zweiten Knochentransplantatelements ermöglicht, wenn das besagte Spreizerelement sich in der besagten Spreizstellung befindet.
